# EUROPEAN PATENT APPLICATION

(11) **EP 4 130 254 A1**
(43) Date of publication of application: **08.02.2023**
(21) Application number: 21189381.3
(22) Date of filing: 03.08.2021
(51) Int. Cl.: C12N 9/00, C12P 21/02

(54) **EUKARYOTIC CELL LYSATES COMPRISING EXOGENOUS ENZYMES AND METHODS FOR PREPARING THE SAME**

(71) Applicant: Fraunhofer-Gesellschaft zur Förderung der angewandten Forschung e.V., 80686 München (DE)
(72) Inventor: Schloßhauer, Jeffrey, 14476 Potsdam (DE); Zemella, Anne, 14476 Potsdam (DE); Kubick, Stefan, 14476 Potsdam (DE); Cavak, Nino, 14476 Potsdam (DE); Thoring, Lena, 14476 Potsdam (DE)
(74) Representative: v. Bezold & Partner Patentanwälte - PartG mbB

(57) **Abstract**

The present invention relates to a method for producing eukaryotic cell lysates comprising at least one exogenous enzyme, wherein the method comprises at least the following steps:
a) providing eukaryotic cells transfected with at least one donor template coding for at least one exogenous enzyme which is selected from the group comprising orthogonal aminoacyl-tRNA synthetases, viral RNA polymerases and kinases;
b) cultivating the transfected cells of step a) for a predetermined period of time and subsequently harvesting the cells; and
c) disrupting the harvested cells and preparing a cell lysate therefrom.

More specifically, the invention relates to a method for producing eukaryotic cell lysates which are capable of cell-free synthesis of a target protein comprising a non-canonical amino acid, wherein the method comprises at least the following steps:
a) providing eukaryotic cells transfected with at least one donor template coding for an orthogonal aminoacyl-tRNA synthetase, i.e. an aminoacyl-tRNA synthetase which is specific for a tRNA which is not recognized by endogenous aminoacyl-tRNA synthetasen in said eukaryotic cells and is specific for a corresponding non-canonical amino acid;
b) cultivating the transfected cells of step a) for a predetermined period of time and subsequently harvesting the cells; and
c) disrupting the harvested cells and preparing a cell lysate therefrom.

Further aspects of the present invention relate to eukaryotic cell lysates obtainable by the claimed method as well as to novel transfected eukaryotic cells or cell lines expressing at least one orthogonal aminoacyl-tRNA synthetase which may be advantageously used in said method.

## Description

### Background of the invention

Nowadays, protein production systems are required for diverse applications ranging from basic research for understanding of complex, cellular mechanisms to the development and production of biotherapeuticals. The demand for improved and novel protein production systems is increasing over the last years aiming for fast and cost-efficient synthesis of complex proteins. In principle protein production can be subdivided into cell-based and cell-free protein synthesis systems. Until now, cell-based systems are well established for research and industrial applications and mostly used for the production of proteins.

While cell-based systems require a time-intensive and laborious modification of living organisms, cell-free protein synthesis systems are a simplified version using cell extracts instead of whole cells. These cell extracts contain all factors required for protein translation including ribosomes, tRNA's, aminoacyl tRNA synthetases and translation factors. The open character makes the system more flexible compared to cell-based production systems. DNA or mRNA encoding the desired target protein can be supplemented directly to the cell-free reaction without the need of elaborate cloning and clone selection steps. Synthesis conditions can be directly adjusted for each individual protein without impairing a cellular function. In recent years various cell-free systems were developed based on eukaryotic and prokaryotic cell-extracts. Prokaryotic cell-free systems lead to high protein yields, but are limited in the performance of posttranslational modifications. Eukaryotic cell-free systems have undergone a tremendous development during the last decades increasing the derived protein yields from low µg/ml up to a mg/ml range of the desired target protein, thereby making them more attractive for protein production. Eukaryotic cell-free systems are typically derived from *Sf*21 cells, CHO cells, human cell lines like HeLa or K562 cells, tobacco cells, yeast cells and reticulocytes.

Eukaryotic systems, in particular those based on *Sf*21, CHO and human cell lysates, enable the production of posttranslational modified proteins. Such post-translational modifications (PTMs) include glycosylation, signal peptide cleavage, phosphorylation, disulfide bond formation and lipid modification. In this context eukaryotic cell-free systems are in particular advantageous for the production of complex mammalian proteins. PTMs can be performed in the presence of so-called microsomes, endogenous membrane particles derived from endoplasmic reticulum. In lysates harboring endogenous microsomes a cotranslational translocation of secreted and membrane embedded proteins takes place mimicking the natural mechanism of ER-based protein processing. This processing typically leads to an increased functionality of complex proteins in contrast to cell-free systems without translocation machinery.

The bottlenecks of cell-free synthesis, especially in eukaryotic cell-free systems, are mostly the costs of the reaction making the cell-free production currently less attractive than cell-based systems for industrial and manufacturing purposes. Currently, most of the cell-free reactions are only performed in small lab-scale reactions and only limited scaled up production platforms are reported for *E.coli* based cell-free systems. Comparing *E.coli* based cell-free systems to eukaryotic cell-free systems, the preparation of lysates is more cost intensive for the eukaryotic ones.

Apart from the cell lysates, additional factors are required to perform an efficient cell-free reaction. To add the template for a desired target protein, DNA encoding the protein can be directly supplemented to the cell-free reaction. According to the central dogma of molecular biology, DNA is transcribed into mRNA before protein translation starts at the ribosomes. To enable mRNA transcription, recombinant RNA polymerases are supplemented to the reaction. In most cases DNA-dependent RNA polymerase from the T7 phage is used to perform the transcription reaction. The supplementation of this recombinant enzyme is one of the most costly issues in a cell-free reaction. Apart from the supplementation of T7 RNA polymerase, further enzymes are required for energy regeneration. To support energy consuming protein synthesis a defined amount of ATP needs to be available. Energy regeneration systems typically consist of an enzyme, which phosphorylates ADP to ATP using a specific substrate. Common energy regeneration systems applied to cell-free protein synthesis are based on the enzymes creatine kinase, pyruvate kinase and acetate kinase. The recombinant kinases are supplemented to the lysate, thereby increasing the productivity of the system by recycling ATP.

Cell-free systems can be further supplemented with orthogonal translation reactants including orthogonal tRNA, non-canonical amino acids (ncaa) and an orthogonal aminoacyl tRNA-synthetase (aaRS). Hence, toxic effects of the non-canonical amino acids in cell-based systems can be overcome and proteins can be modified with defined chemical groups, for instance to modify proteins with fluorescent groups, biopolymers and sugar moieties in a short period of time. Usually, orthogonal aaRSs are added separately to cell-free systems in their purified form, thereby creating additional costs and making it inevitable to implement time consuming purification steps.

T7 RNA polymerase and orthogonal aaRS were previously shown to be expressed from a transformed plasmid in *E.coli* or from an integration site of the *E.coli* genome (US Patent No. 10118950 B2). The subsequent preparation of cell-free extracts is utilized to synthesize proteins in an orthogonal translation reaction. However, such a prokaryotic system is not suitable for the production of complex proteins, since most mammalian proteins require PTMs and an appropriate protein folding apparatus according to eukaryotic reaction conditions. Transfection of mammalian cells and subsequent isolation of positive clones is difficult to implement compared to prokaryotic systems. Prokaryotic cells, such as *E.coli* can be grown faster and are more robust. Furthermore, prokaryotic genetic circuits are well known and can be modified straightforward. In contrast, mammalian protein expression and modification of genomes involves considerably more regulatory factors, which makes it hard to establish a novel production system.

In view of this situation, the main object underlying the present invention is the provision of novel and improved means for cell-free synthesis of proteins, including proteins with posttranslational modifications and and/or non-canonical amino acids, which overcome or considerable alleviate the drawbacks of the prior art, in particular the high costs involved with conventional approaches.

This object is achieved according to the present invention by proving the method of claim 1 for producing eukaryotic cell lysates comprising exogenous enzymes required for cell-free protein synthesis and the eukaryotic cell lysate according to claim 10. Additional aspects and preferred embodiments of the present invention are the subject of further claims.

### Description of the invention

The method of the present invention for producing eukaryotic cell lysates comprising at least one exogenous enzyme according to claim 1 comprises at least the following steps:
a) providing eukaryotic cells transfected with at least one donor template coding for at least one exogenous enzyme which is selected from the group comprising orthogonal aminoacyl-tRNA synthetases, viral RNA polymerases and kinases;
b) cultivating the transfected cells of step a) for a predetermined period of time and subsequently harvesting the cells; and
c) disrupting the harvested cells and preparing a cell lysate therefrom.

Typically, the duration of the cultivation period in step b) for transiently transfected cells is in the range from 2-4 days and for stable transfected cells in the range from 4 to 10 weeks including the stages of single clone selection and expansion of clones. Suitable and optimal cultivation periods can be determined by the skilled artisan by means of routine experimentation.

More specifically, the method of the invention comprises at least the following steps:
a1) transfecting eukaryotic cells with at least one donor template coding for at least one exogenous enzyme which is selected from the group comprising orthogonal aminoacyl-tRNA synthetases, viral RNA polymerases, and kinases;
a2) keeping cells of step a1) on selection pressure by antibiotic selection agents;
a3) selecting single clones of cell pools from a2);
a4) expanding single clones of step a3) to higher cell densities;
a5) analyzing cells of step a4) by genotyping PCR and qPCR and selecting cell clones with the desired target genotype and mRNA expression;
b) cultivating cells of step a5) under controlled conditions, preferably in bioreactors, and subsequent harvesting the cells;
c) disrupting the harvested cells and preparing a cell lysate therefrom.

The term "exogenous enzyme", as used herein, refers to any enzyme which is not naturally produced by the respective eukaryotic cells used in the present invention and which is selected from the group comprising orthogonal aminoacyl-tRNA synthetases, viral RNA polymerases, in particular T7 RNA polymerase, T5 RNA polymerase, and kinases, in particular creatine kinase, pyruvate kinase, acetate kinase. Generally, the presence of these enzymes in a cell lysate is required or at least advantageous for the production of a desired target protein.

In a specific embodiment, the orthogonal aminoacyl-tRNA synthetase is selected from the group comprising pyrrolysine tRNA synthetase, tyrosyl tRNA synthetase and leucine tRNA synthetase. In some more specific embodiments, the orthogonal aminoacyl-tRNA synthetase is *E.coli* tyrosyl-tRNA synthetase, *Methanosarcina mazei* pyrrolysine tRNA synthetase or *E.coli* leucine tRNA synthetase.

A preferred embodiment of the invention relates to a method for producing eukaryotic cell lysates which are capable of cell-free synthesis of a target protein comprising a non-canonical amino acid, wherein the method comprises at least the following steps:
a) providing eukaryotic cells transfected with at least one donor template coding for an orthogonal aminoacyl-tRNA synthetase, i.e. an aminoacyl-tRNA synthetase which is specific for a tRNA which is not recognized by endogenous aminoacyl-tRNA synthetases in said eukaryotic cells and is specific for a corresponding non-canonical amino acid;
b) cultivating the transfected cells of step a) for a predetermined period of time and subsequently harvesting the cells; and
c) disrupting the harvested cells and preparing a cell lysate therefrom.

More specifically, this method comprises at least the following steps:
a1) transfecting eukaryotic cells with at least one donor template coding for an orthogonal aminoacyl-tRNA synthetase;
a2) keeping cells of step a1) on selection pressure by antibiotic selection agents;
a3) selecting single clones of cell pools from a2);
   a4) expanding single clones of step a3) to higher cell densities;
   a5) analyzing cells of step a4) by genotyping PCR and qPCR and selecting cell clones with the desired target genotype and mRNA expression;
   b) cultivating cells of step a5) under controlled conditions, preferably in bioreactors, and subsequent harvesting the cells;
   c) disrupting the harvested cells and preparing a cell lysate therefrom.

The present invention also relates to a method for performing cell-free synthesis of a target protein comprising a non-canonical amino acid, wherein the method comprises at least the following steps:
performing steps a) - c) of the method for producing eukaryotic cell lysates which are capable of cell-free synthesis of a target protein comprising a non-canonical amino acid as outlined above; and step d) incorporating at least one non-canonical amino acid into a target protein in a cell-free protein synthesis reaction based on lysates of step c) comprising at least one orthogonal aminoacyl-tRNA synthetase which is specific for said non-canonic amino acid.

The eukaryotic cells used in the present invention are not especially limited. Principally, any eukaryotic cells used in the prior art for preparing cell extracts are suitable. Preferably such cells are used in the present invention which provide cell lysates containing membrane-vesicles.

Specifically, the eukaryotic cells may be selected from the group which comprises non-human mammalian cells, in particular CHO cells, insect cells, in particular *Spodoptera frugiperda* cells, human cells, in particular HEK293 and K562 cells, and yeasts, in particular *Pichia pastoris* and *Saccharomyces cerevisiae.*

The respective eukaryotic cells are either transiently or stable transfected with the donor template(s) for the desired exogenous protein.

For transient transfection, typically plasmids containing an expression cassette of the respective donor template are introduced in the target cells using PEI (polyethylenimine) as transfection agent using a standard procedure (Rajendra 2011; DOI: 10.1016/j.jbiotec.2011.03.001).

Alternatively, stable transfection is applied to introduce the donor DNA, encoding the protein of interest at a desired genomic locus. For this purpose, the recently established CRISPR/Cas9 (CC9) technology represents an especially efficient and favorable approach for the site-specific incorporation of genes of interest into host cells. A protein complex consisting of the endonuclease Cas9 and a sequence specific RNA molecule can be transfected to cells in combination with a donor template carrying a protein coding sequence of interest. The nuclease guided complex can bind at a desired genomic locus and induce double stranded breaks. During the reparation process the donor sequence can be introduced at the targeted genomic locus by homology directed repair (Ran and Hsu, 2013; DOI: 10.1038/nprot.2013.143). However, other methods for stable transfection of eukaryotic cells known in the art may be used as well.

After a suitable time period, typically two days post-transfection, cells may undergo a selection procedure to enrich edited cells. Afterwards, single cell cloning and expanding of cell clones is performed. Resulting clones are analyzed by genotyping PCR with flanking primers and mRNA expression is monitored by qPCR. A successfully edited cell clone is cultivated under controlled conditions, preferably in a bioreactor, and cells are harvested at optimal growth phases. Cells are disrupted, while keeping endogenous proteins intact for cell-free protein synthesis. Cell lysates are processed by chromatography and supplemented with desired additives. The performance of the newly generated lysate is analyzed by means of a reporter system, which consists of a protein coding sequence harboring an amber stop codon at a defined position and a corresponding reference protein coding sequence without an amber stop codon. Typically, reporter proteins produce either fluorescent or luminescent signals if the amber stop codon is addressed by the orthogonal tRNA coupled to a non-canonical amino acid. The output signal can be compared to the reference protein coding sequence without an amber stop codon

In more specific embodiments, an orthogonal aminoacyl tRNA synthetase, e.g. *E.coli* tyrosyl-tRNA synthetase (eAzFRS), is introduced into the CHO-cells. Such orthogonal synthetases are suitable for site-specific incorporation of non-canonical amino acids into proteins using amber suppression technology. Single cell cloning was performed by a limiting dilution step in 96 well plates. Resulting cell clones were analyzed by genotyping PCR and qPCR. Cell lysates containing the orthogonal eAzFRS are analyzed for amber suppression by utilizing a reporter gene construct, e.g. composed of the membrane protein Adenosine receptor A2a with an introduced amber stop codon position inside the coding sequence and a C-terminal located NanoLuc luciferase. Amber suppression can be further verified by autoradiography on the basis of a higher molecular weight of amber suppressed reporter protein in contrast to terminated protein.

The preparation of cell lysates from the various eukaryotic cells mentioned above is well known in the art (e.g. Brödel, 2013 https://doi.org/10.1002/bit.25013) and, if desired, suitable modifications of known procedures can be effected by the skilled artisan.

The present invention further relates to a eukaryotic cell lysate for cell-free synthesis of a target protein, which cell lysate comprises at least one exogenous enzyme which is required or advantageous for the cell-free synthesis of said target protein and which is selected from the group comprising orthogonal aminoacyl-tRNA synthetases, viral RNA polymerases and kinases.

Preferably the eukaryotic cell lysate is a cell lysate containing membrane-vesicles. Specifically, the eukaryotic cell lysates may be derived from the group of cells which comprises non-human mammalian cells, in particular CHO cells, insect cells, in particular *Spodoptera frugiperda* cells, human cells, in particular HEK293 and K562 cells, and yeasts, in particular *Pichia pastoris* and *Saccharomyces cerevisiae.*

Typically, said eukaryotic cell lysate is capable and used for cell-free synthesis of a complex or specific target protein comprising posttranslational modifications and/or a non-canonical amino acid. Such post-translational modifications (PTMs) include e.g. glycosylation, signal peptide cleavage, phosphorylation, disulfide bond formation and lipid modification.

In one preferred embodiment, said eukaryotic cell lysate comprises at least one orthogonal aminoacyl-tRNA synthetase, i.e. an aminoacyl-tRNA synthetase which is specific for a tRNA not recognized by endogenous aminoacyl-tRNA synthetases in the corresponding eukaryotic cells from which the cell lysate is derived.

Specifically, the orthogonal aminoacyl-tRNA synthetase may be selected from the group comprising pyrrolysine tRNA synthetase, tyrosyl tRNA synthetase and leucine tRNA synthetase.

In some more specific embodiments, the orthogonal aminoacyl-tRNA synthetase is *E.coli* tyrosyl-tRNA synthetase, *Methanosarcina mazei* pyrrolysine tRNA synthetase or *E.coli* leucine tRNA synthetase.

A further aspect of the present invention relates to a transfected eukaryotic cell or cell line expressing at least one orthogonal aminoacyl-tRNA synthetase, wherein the cell is selected from CHO cells, *Spodoptera frugiperda* cells, HEK293 cells, K562 cells, *Pichia pastoris* and *Saccharomyces cerevisiae* and the aminoacyl-tRNA synthetase is selected from *E.coli* tyrosyl-tRNA synthetase, *Methanosarcina mazei* pyrrolysine tRNA synthetase or *E.coli* leucine tRNA synthetase.

### Brief description of the Figures

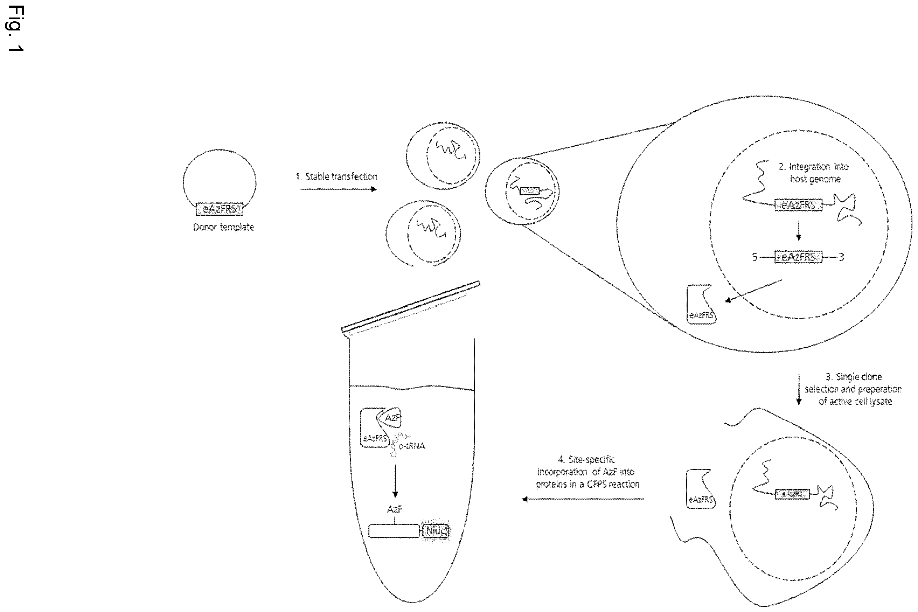

**Figure 1** schematically shows the integration of donor DNA into eukaryotic host cells, followed by preparation of cell lysates and cell-free protein synthesis of a target protein.

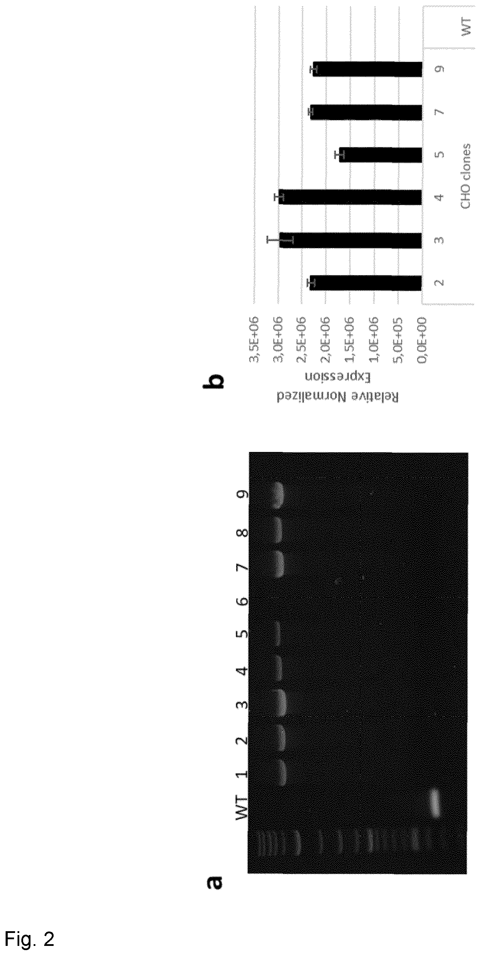

**Figure 2** shows the characterization of selected single CHO clones. Fig.2a: Genotyping of genomically modified CHO cell clones with flanking primer pairs in a PCR reaction. A commercial DNA ladder (0.1-10.0 kb) was used for DNA fragment sizing; Fig. 2b: Estimation of transcriptional activity of CRISPR engineered CHO single clones by qPCR. Normalization of the determined transcriptional expression levels of the eAZFRS was achieved using the expression levels of the housekeeping gene Gnb1.

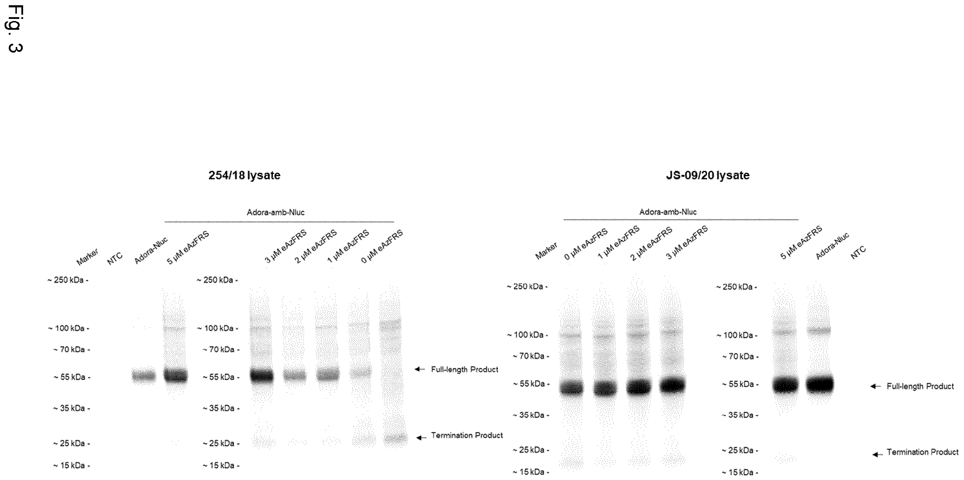

**Figure 3** shows the expression of target proteins in cell lysates derived from a clonal CHO cell line based on CRISPR edited cells. Target proteins could be visualized by autoradiography based on protein size and incorporation of ¹⁴C leucine during cell-free protein synthesis. The autoradiogram corresponds to the Nano-Glo^{®} luciferase assay shown in Table 3.

The present invention is further illustrated by the following specific but non-limiting examples.

### EXAMPLE 1

### Transfection of CHO cells with aminoacyl-t-RNA synthetases

### 1. Transient transfection

Plasmids containing an expression cassette for *E.coli* tyrosyl-tRNA synthetase (eAzFRS) or *Methanosarcina mazei* pyrrolysine tRNA synthetase (pylRS-AF), respectively, were introduced into the CHO host cells using PEI as transfection agent. Therefore, 1x10⁹ cells/ml were pelleted by centrifugation with 200xg for 5min at 4°C. The cell pellets were resuspended with ProCHO5 medium supplemented with 4 mM Ala-Glu in a volume of 25 ml with a cell density of 40x10⁶ cells/ml in a 50 ml conical tube. For transfection 1.5 µg plasmid DNA was added per 1x10⁶ cells. Afterwards 2 µg PEI reagent was added per 1x10⁶ cells. The mixture of transfection reagent, plasmid and cells was incubated for 4 hours at 37°C, 5% CO2 and rotated on a wheel. Subsequent dilution of cells to a final volume of 1 liter (cell density of 1x10⁶ cells/ml) was carried out with ProCHO5 Medium and 4 mM Ala-Glu. Transfected cells were cultivated under controlled conditions in 1 L bioreactors (and alternatively in 2 L shake flasks) and cells were harvested at optimal growth phases, typically about two days post-transfection.

Cells were disrupted, while keeping endogenous proteins intact for cell-free protein synthesis. Cell lysates were processed by chromatography and supplemented with desired additives. The performance of the newly generated lysates was analyzed by a reporter system (see Table 3 for results).

### 2. Stable transfection

*E.coli* tyrosyl-tRNA synthetase (eAzFRS), was introduced into the CHO-K1 genome according to a modified protocol of Zhao et al. 2018 (DOI: 10.1007/s00253-018-9021-6). The eAzFRS is orthogonal to eukaryotic cells and thereby suitable for site-specific incorporation of non-canonical amino acids into proteins by amber suppression technology. For stable transfection by CRISPR/Cas9 the three plasmids Donor vector (DV), Cas9 expression vector and guide RNA expression vector were transfected in a ratio of 2:2:1 by Lipofectamine LTX (Thermo Fisher Scientific, Inc.). Therefore, 5 µl with 500 ng plasmid mixture was added to 100 µl Opti-MEM reduced medium, followed by addition of 2.5 µl Lipofectamine LTX (Thermo Fisher Scientific, Inc.) and 0.5 µl PLUS reagent (Thermo Fisher Scientific, Inc). The DNA-lipid mixture was incubated for 30 min at room temperature. Afterwards the 100 µl of the mixture was added to 500 µl with 0.5x10⁶ cells in a 24 well plate. The plate was centrifuged at 400xg for 15 min at room temperature to increase the transfection efficiency. Cells were incubated at 37°C and 5% CO2. Two days post-transfection, cells undergo a selection procedure to enrich edited cells. Therefore, the culture was supplemented with 10 µg/ml puromycin and selection medium was changed twice a week. Single cell cloning was performed by either array dilution method or a limiting dilution step in 96 well plates in a volume of 100 µl and using 50% conditioned medium. Resulting cell clones were expanded and analyzed by genotyping PCR and qPCR. Cells were harvested at optimal growth phases and disrupted to produce cell lysate for cell-free protein synthesis. Cell lysates containing the orthogonal eAzFRS were analyzed for amber suppression by utilizing a reporter gene construct composed of the membrane protein Adenosine receptor A2a with an introduced amber stop codon position inside the coding sequence and a C-terminal located NanoLuc luciferase. Amber suppression was further verified by autoradiography on the basis of a higher molecular weight of amber suppressed reporter protein in contrast to terminated protein.

### EXAMPLE 2

### Characterization of stable transfected CHO cells

DNA coding for eAzFRS had been incorporated into four different target sites of the HPRT or C12orf35 locus of the CHO-K1 genome as outlined in Example 1. The green fluorescent protein (GFP) sequence was further integrated into the genome as a reporter gene to proof the gene editing efficiency of the gene of interest (Gol) utilizing the CRISPR/Cas9 (CC9) HDR technology for each sgRNA:DV pair.

The array dilution method was utilized to cultivate CHO cells in different cell densities, while keeping selection pressure with 10 µg/ml puromycin constant. Positively transfected clone pools were gradually expanded from 96- well to 12- well plates to achieve an higher amount of cells. Afterwards cells were analyzed by qPCR.

### 1. Transcriptional analysis of CHO clone pools

Two CHO clone pools, each transfected with a donor vector (DV) carrying either the coding sequence of eAzFRS or GFP, were selected based on growth behavior for qPCR analysis as shown in Tab. 1.

QPCR was carried out with primer sequences specific for the target genes and for the house keeping genes (HKGs) Gnb1 and Fkbp1A. The transcriptional expression was normalized to the HKGs and visualized as ratio of Gnb1/Fkbp1A.

**Table 1: Comparison of the transcriptional expression levels of eAzFRS and GFP in stably CRISPR engineered CHO cells. qPCR measurements were performed in triplicate.**

| **Target** | **Clone pool** | **Ratio Gnb1 +FkBp1A** | **Standard deviation** |
|---|---|---|---|
| AzFRS | DV5.1 | 4,902 | 0,748 |
| | DV5.2 | 9,888 | 1,575 |
| | DV6.1 | 13,696 | 0,942 |
| | DV6.2 | 2,408 | 0,484 |
| | DV7.1 | 13,805 | 1,768 |
| | DV7.2 | 27,334 | 2,125 |
| | DV8.1 | 4,878 | 0,185 |
| | DV8.2 | 37,742 | 4,975 |
| | Untreated | 0,096 | 0,028 |
| GFP | DV9.1 | 26,933 | 2,354 |
| | DV9.2 | 24,147 | 1,997 |
| | DV10.1 | 18,697 | 1,685 |
| | DV10.2 | 25,218 | 2,751 |
| | DV11.1 | 25,399 | 0,673 |
| | DV11.2 | 20,406 | 0,940 |
| | Untreated | 0,016 | 0,003 |

Table 1 displays the differences of isolated clone pools based on various sgRNA:DV pairs based on the transcription profile. Untreated CHO cells were utilized as negative control to show the absence of the target gene sequence of the CHO-K1 cell line. CHO clone pools based on DV 7.2 and DV 8.2 reached highest values of 27.3 and 37.7, respectively. In contrast, clone pools based on DV 5 and DV 6 achieve values up to 13.7. Thus, transcriptional activity of the eAzFRS gene seems to be higher utilizing DV addressing the C12orf35 locus (DV 7 and DV8) compared to DV with homology to the HPRT locus. However, stable transfection with GFP showed similar values for CHO clone pools based on targeting of the HPRT locus (DV 9 and DV 10) and C12orf35 locus (DV 11) ranging from 18.7 (DV 10.1) to 26.9 (DV 9.1), indicating that the targeted loci do not significantly differ in transcriptional activity. Indeed, clone pool DV 8.2 showed the strongest mRNA expression level of all tested clone pools and was thus subjected to single cell cloning.

### 2. Characterization of CHO single cell clones

Successful incorporation of the cloning cassettes inside the desired locus of the single clones was verified by genotyping-PCR. Genotyping primers bind specifically down- and upstream of the integration site in the C12orf35 locus of the CHO genome. Fig. 2 shows the characterization of selected single clones. Fig.2a: Genotyping of genomically modified CHO cell clones with flanking primer pairs in a PCR reaction. A Quick-Load^{®} 2-Log DNA Ladder (0.1-10.0 kb) from New England Biolabs was used for DNA fragment sizing. Due to the large incorporated DNA sequence, a higher product size for single clones could be detected, while a small product was visualized for wild type CHO cells (WT). Fig. 2b and Table 2 depict the transcriptional activity of CRISPR engineered CHO single clones by qPCR. Normalization of the determined transcriptional expression levels of the eAZFRS was achieved using the expression levels of the housekeeping gene Gnb1. qPCR measurements were performed in triplicates.

**Table 2: transcriptional activity of CRISPR engineered CHO single clones**

| **Clone** | **Ratio Gnb1** | **Standard deviation** |
|---|---|---|
| 2 | 8,26 | 0,26 |
| 3 | 9,701 | 1,628 |
| 4 | 9,865 | 0,561 |
| 5 | 5,868 | 0,346 |
| 7 | 8,84 | 0,528138065 |
| 9 | 8,208 | 0,290 |
| | 0,000 | 0,000 |

In case of single clone 6, the expression cassette was either removed by cells or CHO cells, which were not specifically modified at the desired locus survived the selection process by random integration of the puromycin gene. Indeed, single clones 1 and 8 displayed integration of the target sequence, but grew only slowly (Fig. 2b). Nonetheless, single clones 2-5, 7 and 9 achieved higher growth rates and were subjected to the analysis of mRNA content. While elevated mRNA expression levels are observed for 3 and 4, single clone 7 was utilized for further crude extract preparation, since it shows highest growth rates.

Cultivation of the clonal CHO cell line was performed in a 1 L scale bioreactor prior to crude extract preparation. The cultivation process was controlled by utilizing critical parameters including pO2, pH, stirrer speed and temperature. During cultivation following set points were used to control the CHO fermentation process: temperature: 37°C, pO2: 40% and pH: 7.1. The setpoint of pO2 was maintained by applying a pO2 regulation cascade, depending on stirring, O₂ and air. The increasing viable cell number was monitored over approximately 9 days, starting from ∼5 × 10⁵ cells/mL. Cells were harvested for the subsequent cell disruption at densities of 4-6 × 10⁶ cells/mL, after 3 days, while keeping a cell density of ∼5 × 10⁵ cells/mL in the bioreactor for further cultivation over 3 days. This so-called repeated batch was carried out over 9 days with 3 harvest points for crude extract preparation for cell-free protein synthesis.

Typically, cell viability ranged from 95 % up to 100 % throughout the whole cultivation process, indicating optimal conditions for preparation of translationally active cell lysates.

### EXAMPLE 3

### Evaluation of cell lysates derived from CHO cells transfected with eAzFRS

Orthogonal translation with the newly generated cell lysates from different harvest points of the repeated batch method, was characterized to analyze the stability and comparability of the cell lysates (data not shown). The novel generated cell lysate based on clone 7 and harvest point 3 referred as to "JS-09/20" was compared to the reference lysate 254/15. Cell lysate prepared from transiently transfected CHO cells referred to as "AzFRS lysate" was further compared to the reference lysate.

**Table 3:**

| **Transfection method** | **Cell lysate** | **Template** | **Concentration of supplemented eAzFRS [µM]** | **Relative luminescent units [RLU]** | **Standard deviation** |
|---|---|---|---|---|---|
| Stable transfection | JS-09/20 | Adora-amb-Nluc | 0 µM | 1,14E+06 | 3,67E+04 |
| | | | 1 µM | 1,11E+06 | 4,19E+04 |
| | | | 2 µM | 1,33E+06 | 4,08E+04 |
| | | | 3 µM | 1,38E+06 | 2,72E+04 |
| | | | 5 µM | 1,44E+06 | 1,29E+04 |
| | | Adora-Nluc | | 2,94E+06 | 7,63E+04 |
| | | NTC | | 1,13E+02 | 1,12E+01 |
| | 254/18 | Adora-amb-Nluc | 0 µM | 1,39E+04 | 3,30E+02 |
| | | | 1 µM | 2,15E+05 | 1,02E+03 |
| | | | 2 µM | 5,05E+05 | 8,34E+03 |
| | | | 3 µM | 5,37E+05 | 1,61 E+04 |
| | | | 5 µM | 8,02E+05 | 2,01E+04 |
| | | Adora-Nluc | | 1,20E+06 | 4,02E+04 |
| | | NTC | | 1,37E+02 | 4,24E+00 |
| Transient transfection | AzFRS lysate | Adora-amb-Nluc | 0 µM | 1,28E+06 | 1,19E+05 |
| | PEI lysate | | | 3,90E+04 | 2,27E+03 |
| | 254/18 | | | 2,10E+04 | 7,30E+02 |
| | AzFRS lysate | | 3 µM | 1,20E+06 | 5,70E+04 |
| | PEI lysate | | | 1,16E+06 | 5,36E+04 |
| | 254/18 | | | 7,47E+05 | 2,51E+03 |
| | | NTC | | 3,00E+01 | 5,20E+00 |
| | | Adora-Nluc | | 1,53E+06 | 8,28E+04 |

Tab. 3 shows the expression of target proteins in cell lysates derived from transiently transfected cells and CRISPR-Cas9 modified CHO clones: Amber suppression on position 215 was utilized in CFPS to express the full-length fusion-protein Adora 2a-amb/Nluc. Adora 2a/Nluc without amber stop-codon was utilized as a positive control. Determination of luciferase activity estimated by utilizing Nano-Glo^{®} luciferase assay (Promega) in a luminescence plate reader (Mithras). Measurements were performed intriplicate. Fig. 3 depicts the corresponding autoradiography to the Nluc-assay of the stable transfection method shown in Tab. 3.

The C-terminal Nluc-activity of Adora2a-amb-Nluc produced 1.1*10⁶ RLU without the addition of further eAzFRS, while no activity was observed for the NTC in case of the stable transfection method (Tab. 2). The transient transfection method displays ∼ 1.3*10⁶ RLU. Indeed, cells treated only with PEI show low values of ∼ 0.04*10⁶ RLU, which is higher than the NTC, but the reference lysate without addition of eAzFRS show a similar low Nluc activity with a RLU of ∼ 0.02*10⁶ . The results suggest that AzF could besite-specifically introduced into the G protein-coupled receptor with the new lysates.

Furthermore, higher concentrations of eAzFRS were supplemented to cell-free reactions based on the JS-09/20 and reference lysate with only minor effects of the CRISPR based cell lysate, while supplementation of eAzFRS significantly increase Nluc-activity in cell-free reaction with the reference lysate. These results could be verified by corresponding band intensities in the SDS-PAGE gel based on autoradiography (Fig. 3).

## Claims

1. A method for producing eukaryotic cell lysates comprising at least one exogenous enzyme, wherein the method comprises at least the following steps:
a) providing eukaryotic cells transfected with at least one donor template coding for at least one exogenous enzyme which is selected from the group comprising orthogonal aminoacyl-tRNA synthetases, viral RNA polymerases and kinases;
b) cultivating the transfected cells of step a) for a predetermined period of time and subsequently harvesting the cells; and
c) disrupting the harvested cells and preparing a cell lysate therefrom.

2. The method according to claim 1 for producing eukaryotic cell lysates which are capable of cell-free synthesis of a target protein comprising a non-canonical amino acid, wherein the method comprises at least the following steps:
a) providing eukaryotic cells transfected with at least one donor template coding for an orthogonal aminoacyl-tRNA synthetase, i.e. an aminoacyl-tRNA synthetase which is specific for a tRNA which is not recognized by endogenous aminoacyl-tRNA synthetasen in said eukaryotic cells and is specific for a corresponding non-canonical amino acid;
b) cultivating the transfected cells of step a) for a predetermined period of time and subsequently harvesting the cells; and
c) disrupting the harvested cells and preparing a cell lysate therefrom.

3. The method of claim 1, comprising at least the following steps:
a1) transfecting eukaryotic cells with at least one donor template coding for at least one exogenous enzyme which is selected from the group comprising orthogonal aminoacyl-tRNA synthetases, viral RNA polymerases, and kinases;
a2) keeping cells of step a1) on selection pressure by antibiotic selection agents;
a3) selecting single clones of cell pools from a2);
a4) expanding single clones of step a3) to higher cell densities;
a5) analyzing cells of step a4) by genotyping PCR and qPCR and selecting cell clones with the desired target genotype and mRNA expression;
b) cultivating cells of step a5) under controlled conditions, preferably in bioreactors, and subsequent harvesting the cells;
c) disrupting the harvested cells and preparing a cell lysate therefrom.

4. The method of claim 2, comprising at least the following steps:
a1) transfecting eukaryotic cells with at least one donor template coding for an orthogonal aminoacyl-tRNA synthetase;
a2) keeping cells of step a1) on selection pressure by antibiotic selection agents;
a3) selecting single clones of cell pools from a2);
a4) expanding single clones of step a3) to higher cell densities;
a5) analyzing cells of step a4) by genotyping PCR and qPCR and selecting cell clones with the desired target genotype and mRNA expression;
b) cultivating cells of step a5) under controlled conditions, preferably in bioreactors, and subsequent harvesting the cells;
c) disrupting the harvested cells and preparing a cell lysate therefrom.

5. A method for performing cell-free synthesis of a target protein comprising a non-canonical amino acid, wherein the method comprises at least the following steps:
performing steps a) - c) of the method according to claim 2 or 4; and
step d) incorporating at least one non-canonical amino acid into a target protein in a cell-free protein synthesis reaction based on lysates of step c) comprising at least one orthogonal aminoacyl-tRNA synthetase which is specific for said non-canonic amino acid.

6. The method according to any one of claims 1-5, wherein the eukaryotic cells are selected from the group which comprises non-human mammalian cells, in particular CHO cells, insect cells, in particular *Spodoptera frugiperda* cells, human cells, in particular HEK293 and K562 cells, and yeasts, in particular *Pichia pastoris* and *Saccharomyces cerevisiae.*

7. The method according to any one of claims 1-6, wherein the eukaryotic cell lysate contains membrane vesicles.

8. The method according to any one of claims 1-7, wherein the orthogonal aminoacyl-tRNA synthetase is selected from the group comprising pyrrolysine tRNA synthetase, tyrosyl tRNA synthetase and leucine tRNA synthetase.

9. The method according to claim 8, wherein the orthogonal aminoacyl-tRNA synthetase is *E.coli* tyrosyl-tRNA synthetase, *Methanosarcina mazei* pyrrolysine tRNA synthetase or *E.coli* leucine tRNA synthetase.

10. A eukaryotic cell lysate for cell-free synthesis of a target protein, which cell lysate comprises at least one exogenous enzyme which is required or advantageous for the cell-free synthesis of said target protein and which is selected from the group comprising orthogonal aminoacyl-tRNA synthetases, viral RNA polymerases and kinases.

11. The eukaryotic cell lysate according to claim 10 for cell-free synthesis of a target protein comprising posttranslational modifications and/or a non-canonical amino acid.

12. The eukaryotic cell lysate according to claim 10 or 11, which cell lysate comprises at least one orthogonal aminoacyl-tRNA synthetase, i.e. an aminoacyl-tRNA synthetase which is specific for a tRNA not recognized by endogenous aminoacyl-tRNA synthetasen in the corresponding eukaryotic cells from which the cell lysate is derived.

13. A transfected eukaryotic cell or cell line expressing at least one orthogonal aminoacyl-tRNA synthetase, wherein the cell is selected from CHO cells, *Spodoptera frugiperda* cells, HEK293 cells, K562 cells, *Pichia pastoris* and *Saccharomyces cerevisiae* and the aminoacyl-tRNA synthetase is selected from *E.coli* tyrosyl-tRNA synthetase, *Methanosarcina mazei* pyrrolysine tRNA synthetase or *E.coli* leucine tRNA synthetase.
